(19) Europäisches Patentamt
European Patent Office
Office européen des brevets

(11) Numéro de publication: **0 465 294 B1**

(12) **FASCICULE DE BREVET EUROPEEN**

(45) Date de publication de fascicule du brevet: **26.10.94**

(51) Int. Cl.5: **C07C 17/16**

(21) Numéro de dépôt: **91401686.0**

(22) Date de dépôt: **21.06.91**

(54) **Procédé de fabrication de bromures insaturés.**

(30) Priorité: **05.07.90 FR 9008559**

(43) Date de publication de la demande:
**08.01.92 Bulletin 92/02**

(45) Mention de la délivrance du brevet:
**26.10.94 Bulletin 94/43**

(84) Etats contractants désignés:
**AT BE CH DE DK ES FR GB GR IT LI LU NL SE**

(56) Documents cités:
**FR-A- 874 166**

**HOUBEN-WEYL: "Methoden der organischen Chemie", édition 4, vol. V/4: "Halogenverbindungen", 1960, pages 377-382, Georg Thieme Verlag, Stuttgart, DE**

(73) Titulaire: **ELF ATOCHEM S.A.**
**4 & 8, Cours Michelet**
**La Défense 10**
**F-92800 Puteaux (FR)**

(72) Inventeur: **Decaudin, Robert**
**27 Rue Foche**
**F-13330 Pelissanne (FR)**
Inventeur: **Reynaud, Jean-Louis**
**No 1, Lotissement Le Garrigo,**
**Le Collet**
**F-13180 Gignac la Nerthe (FR)**

EP 0 465 294 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de fabrication de bromures insaturés par réaction de l'acide bromhydrique avec des alcools insaturés. On l'applique avantageusement à la réaction de l'alcool allylique avec l'acide bromhydrique pour préparer le bromure d'allyle.

On a déjà préparé le bromure d'allyle par la simple réaction de l'alcool allylique et de l'acide bromhydrique. Cette synthèse est décrite dans ULMANN'S ENCYCLOPEDIA OF INDUSTRIAL CHEMISTRY, 5ème édition, volume A4, page 413. On peut opérer avec une solution à 60 % d'acide bromhydrique. On obtient une phase aqueuse contenant l'acide bromhydrique en excès et une phase organique dont on récupère par distillation le bromure d'allyle. On récupère aussi du bromure d'allyle par extraction de la phase aqueuse.

Le rendement molaire sur l'alcool est de 81 % réparti en 75 % dans la phase organique et 6 % dans la phase aqueuse. On peut aussi opérer avec une solution d'acide bromhydrique qui contient de l'acide sulfurique. Le rendement sur l'alcool peut atteindre 92 % mais la solution aqueuse résiduaire contenant de l'acide bromhydrique et l'acide sulfurique est difficile à traiter. Dès qu'on la recycle dans le procédé ou qu'on la distille pour en enlever les produits organiques contenus, on constate un important dépôt collant sur les parois des appareils tels que la colonne de distillation et les surfaces de chauffe. On a essayé aussi de distiller les deux phases simultanément (c'est-à-dire sans les avoir séparées). On réussit à obtenir du bromure d'allyle, mais le résidu aqueux génère à la distillation une importante quantité de résidus encrassant notablement l'appareillage utilisé, rendant cette technique inexploitable dans des conditions industrielles normales.

Comme on ne peut pas recycler le résidu aqueux qui contient tout l'acide sulfurique et des matières organiques, le procédé n'est pas utilisable à l'échelle industrielle.

La demanderesse a donc cherché à mettre au point un procédé industries et a ainsi découvert une nouvelle réaction pour préparer des bromures insaturés.

La présente invention concerne un procédé de préparation de bromures insaturés par réaction de l'acide bromhydrique avec des alcools insaturés de formule

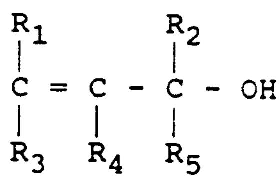

dans laquelle $R_1$, $R_2$, $R_3$, $R_4$ et $R_5$ désignent des atomes d'hydrogène, des alkyls ou des aryls, le nombre total des atomes de carbone de l'ensemble des groupes $R_1$ à $R_5$ étant inférieur ou égal à 25, et de préférence inférieur ou égal à 10, caractérisé en ce qu'on effectue la réaction en présence de cuivre ou d'un sel de cuivre.

On utilise une solution d'acide bromhydrique de préférence aqueuse. La concentration est sans importance mais pour éviter d'utiliser des volumes importants, il est avantageux que la concentration soit d'au moins 300 g par litre, et de préférence entre 400 et 750 g/l. Une concentration supérieure en acide bromhydrique est tout à fait acceptable mais nécessite alors de travailler en réacteur fermé pour éviter les pertes en HBr gazeux.

La quantité d'acide bromhydrique est bien sûr d'au moins une mole HBr par fonction OH à bromer. Il est cependant recommandé d'utiliser un excès d'HBr par rapport à l'alcool pour améliorer à la fois le rendement et le temps de réaction. On utilisera avantageusement un rapport molaire HBr/alcool compris entre 1 et 4, et de préférence voisin de 2.

Il est avantageux d'opérer en présence d'acide sulfurique. On peut utiliser le cuivre ou le sel de cuivre sous forme d'une poudre. On peut utiliser par exemple le chlorure cuivreux, le chlorure cuivrique ou le sulfate de cuivre. La quantité de catalyseur exprimée en métal est comprise entre 0,01 % et 5 % par rapport au poids d'alcool introduit. On emploiera avantageusement une quantité voisine de 0,4 %.

Le rapport entre la quantité d'acide sulfurique exprimée en acide sulfurique $H_2SO_4$ 100 %, et la quantité d'eau présente dans le milieu réactionnel, y compris donc l'eau apportée par l'acide bromhydrique dilué peut être compris entre 0 et 0,6, et de préférence entre 0,25 et 0,5.

Il suffit de mettre en contact l'alcool insaturé, la solution d'acide bromhydrique et le catalyseur. Il est avantageux d'opérer à la pression atmosphérique et à une température comprise entre la température

ambiante et la température d'ébullition du mélange réactionnel.

On ne sortirait pas du cadre de l'invention en opérant sous vide ou sous pression avec une température telle que le milieu réactionnel reste liquide. Il est cependant recommandé de ne pas dépasser 70 °C pour éviter des réactions parasites qui abaisseraient le rendement. Pour avoir une cinétique suffisante de réaction, il est avantageux d'opérer entre 40 et 60 °C, à cette température la durée de réaction est de quelques heures. Il est recommandé d'utiliser un réacteur agité.

A la fin de la réaction, qu'on peut déterminer par la mesure de l'alcool restant dans le milieu réactionnel, on obtient une phase organique contenant essentiellement les bromures insaturés et une phase aqueuse contenant l'acide bromhydrique en excès, et éventuellement l'acide sulfurique. Selon la présente invention, il n'est pas nécessaire de séparer les deux phases puis de les traiter comme dans l'art antérieur pour récupérer les bromures insaturés. On distille les deux phases simultanément, c'est-à-dire non séparées, et on récupère ainsi les bromures insaturés. En fin de distillation il reste une solution aqueuse contenant l'excès d'acide bromhydrique, le catalyseur et éventuellement l'acide sulfurique. Il suffit de recharger cette solution en acide bromhydrique pour la recycler dans une nouvelle synthèse de bromures insaturés selon l'invention.

Le procédé de l'invention permet d'atteindre un rendement molaire en bromures insaturés par rapport à l'alcool d'au moins 95 %, la solution aqueuse peut être recyclée dans la synthèse sans les inconvénients cités précédemment. Par la simple distillation du milieu réactionnel en fin de réaction, on obtient des bromures insaturés avec une pureté de plus de 99,5 % en poids.

Un autre avantage du procédé de l'invention est qu'il n'est pas nécessaire de décanter le milieu réactionnel puis de traiter séparément la phase organique et la phase aqueuse. On peut aussi effectuer le procédé de l'invention en continu.

## EXEMPLE 1

Dans un appareillage comprenant :

- un ballon de 500 ml équipé d'une colonne verre surmontée d'un condenseur,
- un jeu de vannes permettant soit de soutirer le produit condensé, soit de le refluer dans le ballon,
- d'un chauffe-ballon agitateur équipé d'un système de régulation de température.

On introduit :

- 242 ml d'une solution bromhydrique titrant 670 g/l en HBr,
- 90 g d'$H_2SO_4$ à 98 %,
- 58,1 g d'alcool allylique
- 0,25 g de Cu (sous forme $CuSO_4$)

Le mélange, sous agitation et sous reflux total est monté à 60 °C, puis maintenu 2 heures à cette température.

On distille alors, directement à partir du milieu réactionnel, la phase organique sous pression réduite (140 mm de Hg). On obtient un bromure d'allyle tirant 99,66 %, avec un rendement de 94,9 % par rapport à l'alcool introduit. Le réacteur de synthèse ne présente aucun dépôt. La phase acide contient 0,23 g/l de résidu, sans autre présence de produit organique éventuellement distillable à plus haute température.

Un essai similaire, mais sans ajout de cuivre, donne un produit titrant 98,4 % en bromure d'allyle, avec un rendement n'excédant pas 88 %. Le réacteur de synthèse est encrassé (1 % de dépôt par rapport au bromure d'allyle produit). La phase acide, par distillation à pression atmosphérique, génère 3 % de produit organique lourd et contient plus de 1 % de résidu solide (les pourcentages sont exprimés par rapport au bromure d'allyle produit).

## EXEMPLE 2

Dans le même appareillage que dans l'exemple 1, on effectue une réaction d'estérification d'alcool crotylique ($CH_3$-CH = CH-$CH_2$OH par HBr aqueux).

On introduit :

- 72 g d'alcool crotylique,
- 376,3 g d'acide bromhydrique à 670 g/l,
- 96 g d'$H_2SO_4$ 98 %
- dans un cas 0,3 g de Cu (sous forme $CuSO_4$), dans un autre cas pas de cuivre.

Les conditions de synthèse sont identiques à celles de l'exemple 1.

On obtient les résultats suivants.

| Catalyseur ($CuSO_4$) | Rendement par rapport à l'alcool | Résidus en phase acide | Produits obtenus ① | ② |
|---|---|---|---|---|
| Non | 77,45 % | 2,6 g/l | 13,5 | 86,1 |
| Oui | 85,8 % | 0,2 g/l | 14,6 | 84,9 |

① **représente** $CH_3-CHBr-CH = CH_2$
② **représente** $CH_3-CH = CH-CH_2Br$

On constate donc que le catalyseur :

- diminue de façon notable les produits de dégradation formés pendant la synthèse,
- améliore la cinétique (rendement plus élevé pour un temps de synthèse identique)
- n'influe pas sur la sélectivité de la réaction.

## Revendications

**1.** Procédé de préparation de bromures insaturés par réaction de l'acide bromhydrique avec des alcools insaturés de formule

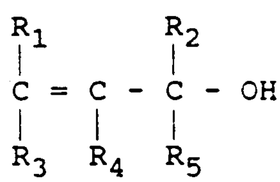

dans laquelle $R_1$, $R_2$, $R_3$, $R_4$ et $R_5$ désignent des atomes d'hydrogène, des alkyls ou des aryls, le nombre total des atomes de carbone de l'ensemble des groupes $R_1$ à $R_5$ étant inférieur ou égal à 25, et de préférence inférieur ou égal à 10, caractérisé en ce qu'on effectue la réaction en présence de cuivre ou d'un sel de cuivre.

**2.** Procédé selon la revendication 1 caractérisé en ce qu'on opère en présence d'acide sulfurique.

**3.** Procédé selon l'une des revendications 1 à 2, caractérisé en ce qu'après la réaction de l'acide bromhydrique et des alcools insaturés, on discille le milieu réactionnel sans avoir préalablement séparé les phases, pour récupérer les bromures insaturés.

## Claims

**1.** Process for the preparation of unsaturated bromides by reaction of hydrobromic acid with unsaturated alcohols of formula

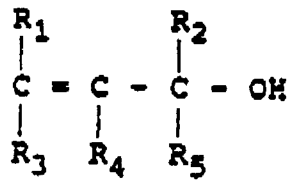

in which $R_1$, $R_2$, $R_3$, $R_4$ and $R_5$ denote hydrogen atoms, alkyls or aryle, the total number of the carbon atoms of the combination of the groups $R_1$ to $R_5$ being smaller than or equal to 25 and preferably smaller than or equal to 10, characterized in that the reaction is carried out in the presence of copper or a copper salt.

**2.** Process according to Claim 1, characterized in that the operation is carried out in the presence of sulphuric acid.

**3.** Process according to either of Claims 1 and 2, characterized in that, after the reaction of hydrobromic acid and of the unsaturated alcohols, the reaction mixture is distilled without the phases having been separated beforehand, to recover the unsaturated bromides.

**Patentansprüche**

**1.** Verfahren zur Herstellung von ungesättigten Bromiden durch Reaktion von Bromwasserstoff mit ungesättigten Alkoholen der Formel

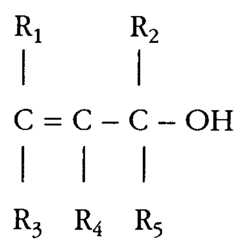

in der $R_1$, $R_2$, $R_3$, $R_4$ und $R_5$ Wasserstoffatome, Alkyl- oder Arylgruppen darstellen, wobei die Gesamtzahl der Kohlenstoffatome der Gruppen $R_1$ bis $R_5$ insgesamt kleiner oder gleich 25, und vorzugsweise kleiner oder gleich 10 ist, dadurch gekennzeichnet, daß die Reaktion in Gegenwart von Kupfer oder einem Kupfersalz durchgeführt wird.

**2.** Verfahren nach Anspruch 1, dadurch gekennzeichnet, daß in Gegenwart von Schwefelsäure gearbeitet wird.

**3.** Verfahren nach Anspruch 1 oder 2, dadurch gekennzeichnet, daß nach Reaktion von Bromwasserstoff mit den ungesättigten Alkoholen eine Destillation des Reaktionsmediums ohne vorherige Trennung der Phasen durchgeführt wird, um die ungesättigten Bromide zurückzugewinnen.